# EUROPEAN PATENT SPECIFICATION

(11) **EP 2 823 423 B1**
(45) Date of publication and mention of the grant of the patent: **13.10.2021**
(21) Application number: 13718904.9
(22) Date of filing: 07.03.2013
(51) Int. Cl.: G16H 50/30, A61B 5/00, A61B 5/11

(54) **GENERATING A CIRCADIAN TIME DIFFERENCE**
ERZEUGUNG EINER ZIRKADIANEN ZEITDIFFERENZ
GÉNÉRATION D'UNE DIFFÉRENCE D'INSTANT CIRCADIEN

(30) Priority: 07.03.2012 US 201261607649 P
(43) Date of publication of application: 14.01.2015
(73) Proprietor: Koninklijke Philips N.V., 5656 AG Eindhoven (NL)
(72) Inventor: PRONK, Serverius Petrus Paulus, NL-5656 AE Eindhoven (NL); MÜSCH, Guido Josef, NL-5656 AE Eindhoven (NL); MAASS, Henning, NL-5656 AE Eindhoven (NL); AUBERT, Xavier Louis Marie Antoine, NL-5656 AE Eindhoven (NL); MOST, Elsa Inger Stapel, NL-5656 AE Eindhoven (NL)
(74) Representative: Philips Intellectual Property & Standards
(86) International application number: PCT/IB2013/051803
(87) International publication number: WO 2013/132454

(56) References cited:
- WO-A1-2008/144908
- US-A- 5 140 562
- US-A- 5 176 133
- US-A1- 2011 144 528
- SMITH M R ET AL: "Phase advancing the human circadian clock with blue-enriched polychromatic light", SLEEP MEDICINE, ELSEVIER, AMSTERDAM, NL, vol. 10, no. 3, 1 March 2009 (2009-03-01), pages 287-294, XP026054061, ISSN: 1389-9457, DOI: 10.1016/J.SLEEP.2008.05.005 [retrieved on 2008-09-19]
- C. MOTT ET AL.: "Model-Based Human Circadian Phase Estimation using a Particle Filter", IEEE TRANSACTIONS ON BIOMEDICAL ENGINEERING, vol. 58, no. 5, 2011, pages 1325-1336, XP11408366, cited in the application
- KANTERMANN T ET AL: "The Human Circadian Clock's Seasonal Adjustment Is Disrupted by Daylight Saving Time", CURRENT BIOLOGY, CURRENT SCIENCE, GB, vol. 17, no. 22, 20 November 2007 (2007-11-20), pages 1996-2000, XP025839620, ISSN: 0960-9822, DOI: 10.1016/J.CUB.2007.10.025 [retrieved on 2007-10-25]
- SANTHI N ET AL: "Scheduling of sleep/darkness affects the circadian phase of night shift workers", NEUROSCIENCE LETTERS, LIMERICK, IE, vol. 384, no. 3, 26 August 2005 (2005-08-26), pages 316-320, XP027653804, ISSN: 0304-3940 [retrieved on 2005-08-26]

## Description

### FIELD OF THE INVENTION

The invention relates to a method of generating information about a person's circadian time cycle for presentation in a graphical user-interface. The invention also relates to a system configured for generating information about a person's circadian time cycle for presentation in a graphical user-interface. The invention further relates to control software, provided as recorded on a computer-readable medium or as an electronic file downloadable over the Internet, for configuring a computer to generate information about a person's circadian time cycle for presentation in a graphical user-interface.

### BACKGROUND ART

In the human being, a misalignment of the person's internal circadian rhythm with the external night/day rhythm can lead to insomnia, delayed sleep-phase disorder (DSPD), (seasonal) affective disorders, etc. The circadian rhythm is controlled by a biological clock in the brain, which has an autonomous rhythm. This biological clock is commonly referred to as the circadian clock. The circadian clock is mainly influenced by light to which the person is being exposed. See, e.g., C.A. Czeisler et al., 1989, "Bright Light Induction of Strong (type-0) Resetting of the Human Circadian Pacemaker", Science 244, pp.1328-1333. A person suffering from above misalignment and seeking medical advice is often prescribed light therapy in order to shift the circadian clock and to better align the person's circadian rhythm with the external night/day rhythm. Diagnostic tools, such as maintaining a sleep log (a sleep diary), or using actigraphy devices or questionnaires, are typically used within the context of light therapy.

The term "actigraphy" refers to the monitoring of a person's rest/activity cycles. Actigraphy typically includes the recording of the movements of the person and of the light exposure levels to which the person is exposed for a number of consecutive days. In particular, data about the light exposure can be used to make an estimation of the circadian phase of the person exposed to the light, in particular when the person's retinae have been exposed to light. The estimation uses a mathematical model of the human circadian clock. At Harvard Medical School (HMS), the circadian clock and the way wherein certain time cues influence the circadian clock have been extensively studied. Exposure to light, and in particular exposure to blue light, are time cues that appear to have the largest impact on the shifting of the circadian clock, as compared to other time cues that affect the circadian clock. The model comprises a set of differential equations, derived from the "van der Pol"-oscillator, well-known in the field of the study of the circadian rhythm.

For more background, see, e.g., R.E. Kronauer et al., 1982, "Mathematical Model of the Human Circadian System with Two Interacting Oscillators", American Journal of Physiology - Regulatory, Integrative and Comparative Physiology 242, R3-R17; R.E. Kronauer, 1990, "A Quantitative Model for the Effects of Light on the Amplitude and Phase of the Deep Circadian Pacemaker, based on Human Data", Proceedings of Sleep'90, pp.306-309; R.E. Kronauer et al., 1999, "Quantifying Human Circadian Pacemaker Response to Brief, Extended, and Repeated Light Stimuli over the Photopic Range", Journal of Biological Rhythms 14:6, pp. 500-515; M.A. St Hilaire et al., 2007, "Addition of a Non-Photic Component to a Light-Based Mathematical Model of the Human Circadian Pacemaker", Journal of Theoretical Biology 247, pp. 583-599; M.A. St Hilaire et al., 2007, "A Physiologically-Based Mathematical Model of Melatonin including Ocular Light Suppression and Interactions with the Circadian Pacemaker", Journal of Pineal Research 43, pp. 294-304; T. Morita etal., 1998, "The Influence of Different Wavelengths of Light on the Human Biological Rhythms", Applied Human Science 17:19; M.E. Jewett et al, 1999, "Revised Limit-Cycle Oscillator Model of the Human Circadian Pacemaker", Journal of Biological Rhythms, Vol. 14(6), pp. 493-500; and C. May et al., 2002, "A Revised Definition of Core Body Temperature Phase that incorporates both State Variables of a Limit-Cycle Human Circadian Pacemaker Model improves Model Stability at low Circadian Amplitude", Abstracts of Society for Research on Biological Rhythms Annual Meeting, p.217.

It is possible to compute an estimation of a person's circadian *phase,* i.e. an arbitrary, but well-defined, reference point in the person's circadian rhythm. The phase can be used to calculate the alignment or misalignment of the circadian rhythm to the external night/day rhythm. Within the field of physiology, the most common reference points are the *wall-clock time* of the dim-light melatonin onset (DLMO) or the *wall-clock time* at which the person's core body temperature (CBT) is minimal, also referred to as the CBT nadir. This estimation can be provided based on actigraphy, i.e., on the recordings of the level of the person's activity and of the level of light exposure. The feature "wall-clock time" refers to the time indicated by an ordinary instrument or time-piece that is used to indicate the time of the day in the time-zone wherein the clock resides, e.g., the time indicated by the clock of the Big Ben clock tower in London, UK.

Information about the temporal character of the circadian phase of a person is useful to a clinician such as a psychiatrist prescribing light therapy. However, this information does not lend itself well for daily use by a layperson, for instance, in the context of self-prescribed light therapy.

Based on the model mentioned above, it is possible to define a notion of *circadian time,* which can be displayed in a manner similar to the familiar *wall-clock time.* In contrast to the wall-clock time, the circadian time does typically not run at a constant rate, but at a varying rate determined by the circadian clock.

US Patent 2011/444528. issued to V. Gurley, discloses a core body temperature (CBT) monitoring system that can be worn by a user and collects continuously CBT data to determine a circadian desynchrony.

US 5,176,133 discloses a method for accurately assessing and rapidly modifying the phase and amplitude of an endogenous circadian pace maker. The endogenous circadian phase may be assessed by employing a regression model to the core body temperature data which has been previously recorded.

US Patent 5,140,562, issued to M.C. Moore-Ede and R.E. Mitchell, discloses the displaying of a person's current circadian time at a timepiece for continuously calculating and displaying the actual biological time of day of an individual. After an initial biological time of the day is entered, the timepiece runs at a pre-determined rate corresponding to the rate at which time would progress in a free-running circadian clock for the individual. When the individual is exposed to clock-altering stimuli (also referred to in the jargon as "Zeitgebers", i.e., "time cues"), such as bright light, the timepiece computes a new operation rate based upon the relative effects of the clock-altering stimuli as determined by a phase-response curve for the individual. By combining information concerning the presence or absence of clock-altering stimuli with information concerning the effects of the stimuli, the watch is able to compute and continuously display the individual's biological time.

A disadvantage of this known timepiece is that the meaning of the biological time as indicated is still insufficiently intuitive to the user. For example, if, at 1:00 pm wall-clock time, the circadian time is displayed as 2:14 pm, it is not clear what this means other than that circadian time is ahead of the current wall-clock time. In particular for applications such as light exposure in the context of shift work, jet-lag relief, treatment of (seasonal) affective disorder, etc., this type of information is not directly usable.

C. Mott et al., 2011, "Model-Based Human Circadian Phase Estimation using a Particle Filter", IEEE Transactions on Biomedical Engineering 58:5, pp. 1325-1336, also relates to presenting the difference between circadian time and wall-clock time, which has disadvantages similar to those of the timepiece of US patent 5,140,562. The approach of Mott et al., is to present a phase-offset relative to the current wall-clock time.

### SUMMARY OF THE INVENTION

The inventors propose ways for presenting the information about a person's circadian time in a more ergonomic way. The ways wherein the information about a person's circadian time is provided render themselves to a more intuitive grasping of the semantic meaning of the circadian time and its usefulness to the person.

To this end, the inventors propose a method according to claim 1 as preferred embodiment of the invention.

In the invention, the person's circadian rhythm is mapped onto basically a two-dimensional domain. One dimension of the domain relates to the circadian time (or: circadian phase) and the other dimension of the domain relates to the wall-clock time. The two-dimensional domain enables to track changes in circadian time by comparing the circadian time at compatible moments of wall-clock time, e.g., wall-clock moments that are 24 hours apart, or 168 hours apart, etc. The two-dimensional domain also enables to track changes in the wall-clock time of the occurrence of a pre-determined circadian phase, e.g., the CBT nadir over a wall-clock time-period of two or more days. Accordingly, the invention enables to visualize the changes. As a person's circadian rhythm will more or less stabilize if the person is exposed to a night/day rhythm and corresponding light exposure that are regular over a longer time-period (e.g., at least a couple of days), a change in either approach indicates to the person a possible discrepancy between his/her night/day rhythm and his/her circadian rhythm.

In a first preferred embodiment of the method of the invention, the method comprises performing a first series of steps as defined in independent claim 1. The specific circadian time is a current circadian time representative of a current circadian phase. The specific wall-clock time is a current wall-clock time coinciding with the occurrence of the current circadian time. Each respective particular past wall-clock time precedes the current wall-clock time by a respective integer number of wall-clock time-periods of 24-hours each. For example, a number of past circadian times which have been sampled at wall-clock moments 24 hours apart, are averaged, possibly using weighting factors, so as to generate the reference circadian time. The current circadian time is matched against the average so as to generate a more stable difference by eliminating day-to-day variations.

In another embodiment of the method of the invention, the method comprises performing the first series of steps as defined in the independent claim and the specific circadian time is a current circadian time representative of a current circadian phase. The specific wall-clock time is a current wall-clock time coinciding with the occurrence of the current circadian time. Each respective particular past wall-clock time precedes the current wall-clock time by a respective integer number of wall-clock time-periods of 168-hours each. The reference circadian time is now derived from the past circadian times occurring at the same wall-clock time of the same day of the week, e.g., Wednesday, over a couple of weeks. The difference is now taking into account the fact that a large number of people lead a life with periods of weekly recurring cycles. By selecting the past circadian times as those of the same day of the week and occurring at the same wall-clock time, variations are reduced that arise from weekly lifestyle fluctuations.

An alternative preferred embodiment of the invention comprises performing a second series of steps as also defined in independent claim 1. The specific circadian phase is representative of a most recent occurrence of a pre-determined phase of the circadian time cycle, e.g., the dim-light melatonin onset (DLMO) or the CBT nadir. The specific wall-clock time is a most recent wall-clock time coinciding with the most recent occurrence of the pre-determined phase. Each respective special past circadian time is representative of a respective past occurrence of the pre-determined phase. In this further embodiment, the occurrence of, e.g., the dim-light melatonin onset (DLMO) or the CBT nadir is tracked over several days, and differences in the wall-clock time of their occurrences over several days (e.g., consecutive days or the same day of consecutive weeks) are visualized

In a further embodiment of the method of the invention, the method comprises the performing of the first series of steps and predicting a future pattern of one or more stimuli that affect the person's circadian time cycle. The first contribution comprises a prediction of a future circadian time occurring at a future wall-clock time compatible with the specific wall-clock time.

In this further embodiment, a prediction is made of the temporal pattern of stimuli that are known to affect a person's circadian rhythm. Examples of such stimuli include light exposure, and activity, as discussed above. A prediction of the temporal pattern of the stimuli can be made if the person has daily routines (e.g., working, commuting, sleeping, sporting, relaxing, shopping, tinkering with old cars, walking the dog, helping the children with their homework, etc.). The routines determine the person's light exposure. A repeated pattern in daily routines is represented in a repeated pattern of daily light exposure and, therefore, in a repeated pattern of the person's circadian rhythm over a time period of, for example, multiple consecutive days. Accordingly, a prediction can be made of the person's circadian time at a future wall-clock time compatible with the specific wall-clock time (e.g., 24 hours later). And this prediction can be visualized as a relative difference relative to the reference circadian time.

Similarly, consider a scenario wherein the method comprises the performing of the second series of steps. The second series of steps may comprise predicting a future pattern of one or more stimuli that affect the person's circadian time cycle. The second contribution comprises a prediction of a future wall-clock time at which a future circadian time occurs compatible with the specific circadian time. For example, given the wall-clock times of the occurrences of the person's CBT nadir in the past up to today, and given the prediction of temporal pattern of stimuli that the person is going to be exposed to tomorrow, a prediction can be made of the wall-clock time tomorrow, at which the person's CBT will occur. The prediction can again be visualized as a difference relative to the reference circadian time.

The invention also relates to a system according to claim 7.

The system in the invention is configured for executing the method specified above. The system may be implemented as, e.g., a device that can be worn by a person. The device has an onboard sensor sub-system to collect information about the person's light exposure, optionally, and the person's activity. The device has a graphical user-interface for presenting the information generated. Alternatively the system may be implemented as spatially distributed. That is, the sensor-sub-system, the memory, the data processor and the graphical user-interface may reside in different geographic locations and communicate with each other via a data network. The person's activity may be taken into consideration in a mathematical model (see, e.g., M.A. St Hilaire et al., 2007, "Addition of a Non-Photic Component to a Light-Based Mathematical Model of the Human Circadian Pacemaker", Journal of Theoretical Biology 247, pp. 583-599, mentioned earlier) or in an adaption of the light exposure. As to the adaption of the light level: when the person is supposed to be in a sleeping state on the basis of the person hardly moving, it may be assumed that the person has his/her eyes closed and that, therefore, his/her retinae are not exposed to light at all.

In an embodiment of the system in the invention, the data processor is configured for performing the first series of steps. The specific circadian time is a current circadian time representative of a current circadian phase. The specific wall-clock time is a current wall-clock time coinciding with the occurrence of the current circadian time. Each respective particular past wall-clock time precedes the current wall-clock time by a respective integer number of wall-clock time-periods of 24-hours each.

In another embodiment of the system of the invention, the data processor is configured for performing the first series of steps and the specific circadian time is a current circadian time representative of a current circadian phase. The specific wall-clock time is a current wall-clock time coinciding with the occurrence of the current circadian time. Each respective particular past wall-clock time precedes the current wall-clock time by a respective integer number of wall-clock time-periods of 168-hours each.

In a further embodiment of the system in the invention, the data processor is configured for performing the second series of steps as defined by claim 1. The specific circadian phase is representative of a most recent occurrence of a pre-determined phase of the circadian time cycle. The specific wall-clock time is a most recent wall-clock time coinciding with the most recent occurrence of the pre-determined phase. Each respective special past circadian time is representative of a respective past occurrence of the pre-determined phase.

In a further embodiment of the system of the invention, the data processor is configured performing of the first series of steps and the first series of steps comprises predicting a pattern, occurring in the future, of one or more stimuli that affect the person's circadian time cycle. The first contribution comprises a prediction of a future circadian time occurring at a future wall-clock time compatible with the specific wall-clock time. Similarly, consider the scenario wherein the data processor is configured for performing of the second series of steps. The second series of steps comprises predicting a pattern, occurring in the future, of one or more stimuli that affect the person's circadian time cycle; and the second contribution comprises a prediction of a future wall-clock time at which a future circadian time occurs compatible with the specific circadian time. The generating of predictions has been discussed above.

The invention also relates to control software according to claim 13, the control software configuring a computer to present information about a person's circadian time cycle at a graphical user-interface, the control software comprising computer instructions to at least perform the steps of the method according to claim 1.

### BRIEF DESCRIPTION OF THE DRAWING

The invention is explained in further detail, by way of example and with reference to the accompanying drawing, wherein:
Fig.1 is a block diagram of a system in the invention; and
Figs. 2, 3, 4, 5 and 6 are diagrams illustrating a visual representation of the information generated in the invention.

Throughout the Figures, similar or corresponding features are indicated by same reference numerals.

### DETAILED EMBODIMENTS

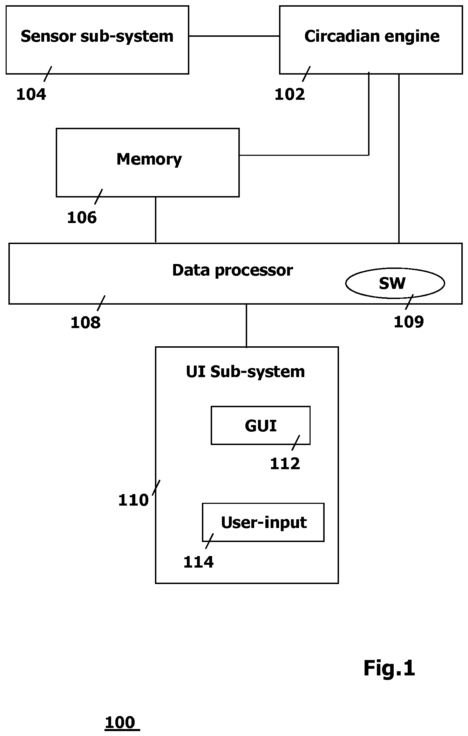

Fig.1 is a block diagram of a system 100 according to the invention. The system 100 comprises a circadian engine 102, a sensor-sub-system 104, a memory 106, a data processor 108 and a user-interface (UI) sub-system 110.

The circadian engine 102 is a module of known functionality, and implemented in hardware, or in software, or in a combination of hardware and software. The circadian engine 102 runs a circadian model of a person. The circadian model comprises, e.g., the circadian model disclosed in M.A. St Hilaire et al., 2007, "Addition of a Non-Photic Component to a Light-Based Mathematical Model of the Human Circadian Pacemaker", Journal of Theoretical Biology 247, pp. 583-599, mentioned above, that has, for example a numerical integrator. The circadian engine 102 receives input from the sensor sub-system 104.

The sensor sub-system 104 comprises one or more light sensors for sensing light incident on the sensor-sub-system. The sensor sub-system 104 supplies light samples to the circadian engine 102. Each respective one of the light samples is representative of the light to which the person is being exposed per respective one of a sequence of time-periods, e.g., the intensity of the light and/or the intensity of the light per interval of wavelength to which the person has been exposed over a single time period. The sensor sub-system 104 also comprises one or more motion sensors for sensing the movements of the person. The sensor sub-system 104 supplies motion samples to the circadian engine 102. Each respective one of the motion samples is representative of a respective activity of the person per respective one of the sequence of time-periods.

Each specific one of the light samples and each specific one of the motion samples comprises a specific time-stamp so as to be able to associate the specific light sample and the specific motion sample with a wall-clock time at which the sensor sub-system 104 was generating the specific light sample and the specific motion sample The specific time-stamp may be added by the sensor sub-system 104 upon generation of the specific light sample and the specific motion sample, or by the circadian engine 102 upon receipt of the specific light sample and the specific motion sample.

As known, once the circadian engine 102 has been initialized and has been synchronized to the person's circadian rhythm, the circadian engine 102 generates respective output data indicative of a respective circadian time (or: circadian phase) of the person under control of the input from the sensor sub-system 104.

The current output data from the circadian engine 102 is stored in the memory 106 together with the current wall-clock time-stamp that ties the current circadian time to the current wall-clock time.

The wall-clock time is kept by, e.g., a clock (not shown) local to the circadian engine 102 or local to the sensor-sub-system 104, or by a clock (not shown) that is external to the circadian engine 102 and external to the sensor sub-system 104, and that sends a wireless clock signal to a receiver (not shown) local to the circadian engine 102 or local to the sensor sub-system 104 so as to enable the circadian engine 102 or the sensor sub-system 104 to add the wall-clock time stamps.

The current output data from the circadian engine 102 is also supplied to the data processor 108.

The data processor 108 is configured to process the current output data from the circadian engine in order to control the UI sub-system 110 for providing information based on the person's current circadian time that is representative of a current circadian phase of the person at the current wall-clock time. For example, the data processor 108 is a general-purpose data processor running dedicated control software 109. The data processor 108 determines the current circadian time from the current output data received from the circadian engine 102. The data processor 108 consults the memory 106 that stores a plurality of past output data from the circadian engine 102. Each respective past output data is representative of respective past circadian time indicative of a respective past circadian phase of the person at a respective past wall-clock time. The data processor 108 selects one or more particular ones of the respective past wall-clock times as will be explained further below, and determines a reference circadian time. The reference circadian time is derived from one or more particular ones of the respective data, representative of one or more particular ones of the respective past circadian times at the one or more particular ones of the respective past wall-clock times selected in the previous step. The data processor 108 then determines a difference between the current circadian time and the reference circadian time, and generates the information under control of the difference, e.g., for presentation in a graphical user-interface (GUI) 112 of the UI sub-system 110.

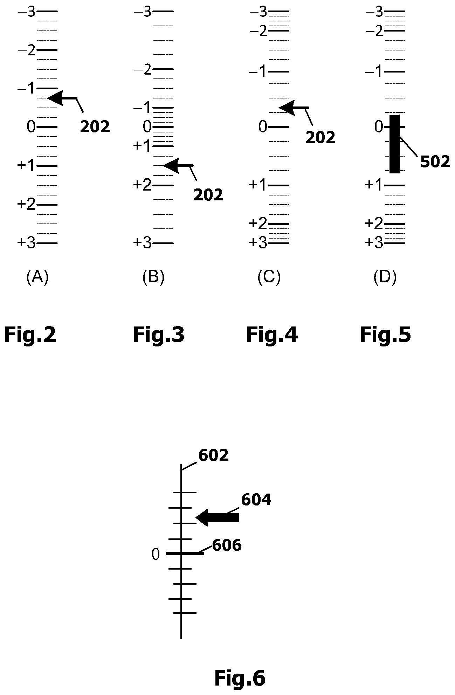

Figs. 2, 3, 4 and 5 are diagrams of examples of information that is generated and presented in the GUI 112 for notifying the person of his/her circadian time. Once the circadian engine 102 has been initialized, synchronized and has been running for at least one day, the difference between the current circadian time and the circadian time exactly one wall-clock day earlier (i.e., 24 hours earlier) is displayed in the GUI 112. The past circadian time, registered in the memory 106 the preceding day at the same wall-clock time as the current wall-clock time, is retrieved from the memory 106. Depending on how long the circadian engine 102 has been running, the past circadian time of N x 24 hours of wall-clock time earlier, may be used to derive a reference circadian time by taking an average of these past circadian times. For example, the reference circadian time is determined based on averaging the N = 5 most recent past circadian times, to wit, the past circadian times of 24 hours ago, 48 hours ago, 72 hours ago, 96 hours ago and 120 hours ago. The average may be a weighted average, for example, giving more weight to the circadian times of the recent past than to the circadian times of longer ago. By means of using the average of two or more past circadian times, the difference between the current circadian time and the reference circadian time is rendered more stable than in case only the past circadian time of 24 hours earlier is used.

Thus, the difference between the current circadian time and the reference circadian time is presented in the GUI 112. The difference is represented, for example, on a one-dimensional scale that enables the person to see in one glance a magnitude as well as a polarity of the difference. The one-dimensional scale comprises a set of calibrated numerical markings against which a pointer moves.

In the diagram of Fig.2, the GUI 112 presents a first scale (A) that is linear. The linear scale (A) shows magnitudes of a positive polarity and magnitudes of a negative polarity, and is centered on a zero magnitude (a reference point). A difference with a positive polarity indicates an advance of the current circadian time relative to the past circadian time of 24 hours ago, or relative to the reference circadian time as discussed above. A difference with a negative polarity indicates a delay, relative to the past circadian time at the same wall-clock time of the previous day, or relative to the reference circadian time. A pointer 202 indicates a position along the first scale (A) that is representative of the difference between the current circadian time and the past circadian time of 24 hours ago, or the difference between the current circadian time and an average of the past circadian times of 24 hours ago, 48 hours ago, 72 hours ago, etc. The arrow 202 indicates in the diagram of Fig.2 an advance of the current circadian time of about ¾ of a wall-clock hour. For clarity it is remarked here that an hour may be taken as a unit of circadian time as well as of wall-clock time. Both the circadian clock and the wall-clock count periods of 24 hours. However, the circadian clock generally runs at a variable rate as compared to the wall-clock. One hour of circadian time is generally not the same as one hour of wall-clock time, and the duration of one hour of circadian time varies in terms of wall-clock time.

The diagram of Fig.3 illustrates a second scale (B), here an expanding scale that can be used in order to be able to emphasize differences of higher magnitude more than differences of lower magnitude. That is, a distance along the expanding scale (B) between successive calibrated numerical marks increases with increasing magnitude of the calibrated numerical marks.

The diagram of Fig.4 illustrates a third scale (C), here a contracting scale that can be used in order to be able to emphasize differences of lower magnitude more than differences of higher magnitude. That is, a distance along the contracting scale (C) between successive calibrated numerical marks decreases with increasing magnitude of the calibrated numerical marks. Different scales may be used in order to depict larger differences. This is especially relevant when considering jet-lag.

In jet-lag applications, circadian phase advances or circadian phase delays occur as a result of travelling and in dependence on the direction of travel. A person will experience an advance in circadian phase with regard to the wall-clock time at his/her geographical position after having travelled west by plane. The person will experience a delay in his/her circadian phase with regard to the wall-clock time at his/her geographical location after having travelled east by plane. In order to help the person adapt to the new time zone at his/her destination the person may be guided by the information presented in the GUI 112. For example, various one-dimensional scales may be combined or another pointer (not shown), e.g., of another color, may indicate a desired shift in circadian phase.

The diagram of Fig.5 illustrates a fourth scale (D) as a variation on the contracting scale of the diagram in Fig.4. The fourth scale (D) is a contracting scale and uses a so-called error bar 502. The error bar 502 visualizes an uncertainty in the difference between the current circadian time and the past circadian time of 24 hours ago, or between the current circadian time and the reference circadian time. The error bar 502 covers a time interval wherein the difference is expected to lie. This uncertainty may stem from an instantaneous uncertainty because of limitations in the numerical model to make accurate estimation of circadian time. Alternatively, or in addition, the uncertainty could also be used to indicate a measure of the day-to-day variability in the person's circadian phase, or a standard deviation relating to the averaging process as described earlier. Alternatively, the error bar 502 is used to indicate the day-to-day variability of the circadian phase, only if the circadian phase as determined by the circadian engine 102 is, e.g., larger than an estimation uncertainty inherent in the numerical model, or outside predefined limits, and the pointer 202 is shown otherwise instead.

In this way, a steady circadian time that exhibits a regular course will give rise to differences of relatively small magnitude, whereas a steadily adapting circadian time may give rise to daily differences of significant magnitude and with positive or negative polarity, depending on the direction of adaptation.

Above examples illustrate the displaying of the difference between the current circadian time and the past circadian time of one or more successive days ago. An alternative is to display the difference between the current circadian time and the past circadian time of exactly one week ago (i.e., of exactly 168 hours ago); or the difference between the current circadian time and a reference circadian time formed by an average of the past circadian time of exactly one week ago (i.e., of exactly 168 hours ago), the past circadian time of exactly two weeks ago (i.e., of exactly 336 hours ago), the past circadian time of exactly three weeks ago (i.e., of exactly 504 hours ago), etc. In this manner, day-to-day variations are eliminated which occur as a result of the person's having working days and non-working days, or as a result of the person's regular activities having a weekly pattern that varies widely from day to day. Other arrangements may be implemented, for instance, based on an 8-day week.

Fig.6 is a diagram illustrating another example of information that is generated in the graphical user interface (GUI) 112 of the UI sub-system 110 for notifying the person of his/her circadian phase. In the diagram of Fig.6, the GUI 112 presents a vertical axis 602 and an arrow 604 positioned relative to the vertical axis 602. The vertical axis 602 has a suitable calibration with a point of reference 606 indicated by "0" or "zero". The point of reference 606 is a reference indicative of an average or of a weighted average of one or more past wall-clock times at which the CBT nadir of the person occurred in the past. If only a single past wall-clock time is considered at which the CBT nadir occurred, the average equals the single wall-clock time. The position of the arrow 604 relative to the point of reference 606 is then indicative of a discrepancy between the wall-clock time of the occurrence of the most recent CBT nadir and the (weighted) average of the past wall-clock time(s). The discrepancy has a positive polarity if the arrow 604 is positioned above the point of reference 606 (as shown in the diagram of Fig.6), and a negative polarity if the arrow 604 is positioned below the point of reference 706. The calibration of the vertical axis 602 may be uniform, as in the diagram of Fig.6, similarly to the calibration in the diagram of Fig.2. Alternatively, the calibration may be non-uniform, similarly to the calibration in the diagrams of Figs. 3-5. Instead of an arrow 604, an error bar (not shown) may be used, similarly to the error bar 502 in the diagram of Fig.5.

## Claims

1. A method of presenting information about a person's circadian time cycle at a graphical user-interface (112), the method comprising:
determining, based on data from one or more light sensors and one or more motion sensors, a specific circadian time of the person that is representative of a specific circadian phase of the circadian time cycle;
determining a specific wall-clock time coinciding with an occurrence of the specific circadian time;
using a memory (106) that stores one or more data items, each respective one of the one or more data items is representative of a respective past circadian time of the person and is representative of a respective past wall-clock time coinciding with a respective past occurrence of the respective past circadian time;
before presenting the information on a graphical user interface, performing at least one of:
a first series of steps that comprises:
selecting from the memory one or more first particular ones of the one or more data items, each respective one of the one or more first particular data items being representative of a respective particular past wall-clock time that precedes the specific wall-clock time by a respective integer number of wall-clock time-periods of 24-hours ;
for each respective one of the one or more first particular data items, determining a respective particular past circadian time;
determining a reference circadian time from the one or more particular past circadian times; and
generating a first contribution to the information based on a difference between the specific circadian time and the reference circadian time;
a second series of steps that comprises:
selecting from the memory one or more second particular ones of the one or more data items, each respective one of the one or more second particular data items being representative of a respective second particular past circadian time at which the circadian time cycle had the same specific circadian phase as the specific circadian time;
for each respective one of the one or more second particular data items, determining the respective second particular past wall-clock time; and
generating a second contribution to the information based on a respective difference between the respective second particular wall-clock time and the specific wall-clock time.

2. The method of claim 1, wherein
the method comprises performing the first series of steps;
the specific circadian time is a current circadian time representative of a current circadian phase;
the specific wall-clock time is a current wall-clock time coinciding with the occurrence of the current circadian time; and
each respective particular past wall-clock time precedes the current wall-clock time by a respective integer number of wall-clock time-periods of 24-hours each.

3. The method of claim 1, wherein
the method comprises performing the first series of steps;
the specific circadian time is a current circadian time representative of a current circadian phase;
the specific wall-clock time is a current wall-clock time coinciding with the occurrence of the current circadian time; and
each respective particular past wall-clock time precedes the current wall-clock time by a respective integer number of wall-clock time-periods of 168-hours each.

4. The method of claim 1, wherein:
the method comprises performing the second series of steps;
the specific circadian phase is representative of a most recent occurrence of a pre-determined phase of the circadian time cycle;
the specific wall-clock time is a most recent wall-clock time coinciding with the most recent occurrence of the pre-determined phase; and
each respective second particular past circadian time is representative of a respective past occurrence of the pre-determined phase.

5. The method of claim 1, wherein:
the method comprises the performing of the first series of steps;
the first series of steps comprises predicting a future pattern of one or more stimuli that affect the person's circadian time cycle;
the first contribution comprises a prediction of a future circadian time occurring at a future wall-clock time compatible with the specific wall-clock time.

6. The method of claim 1, wherein:
the method comprises the performing of the second series of steps;
the second series of steps comprises predicting a future pattern of one or more stimuli that affect the person's circadian time cycle;
the second contribution comprises a prediction of a future wall-clock time at which a future circadian time occurs compatible with the specific circadian time.

7. A system (100) configured for presenting information about a person's circadian time cycle at a graphical user-interface (112), the system comprising:
a circadian engine (102), implemented in hardware and/or software, configured for determining, based on data from one or more light sensors and one or more motion sensors, a specific circadian time of the person that is representative of a specific circadian phase of the circadian time cycle;
a clock configured for determining a specific wall-clock time coinciding with an occurrence of the specific circadian time;
a memory (106) for storing one or more data items, each respective one of the one or more data items is representative of a respective past circadian time of the person and is representative of a respective past wall-clock time coinciding with a respective past occurrence of the respective past circadian time; and
a data processor (108) configured for performing at least one of:
a first series of steps that comprises:
selecting from the memory one or more first particular ones of the one or more data items, each respective one of the one or more first particular data items being representative of a respective particular past wall-clock time that precedes the specific wall-clock time by a respective integer number of wall-clock time-periods of 24-hours;
for each respective one of the one or more first particular data items, determining a respective particular past circadian time;
determining a reference circadian time from the one or more particular past circadian times; and
generating a first contribution to the information based on a difference between the specific circadian time and the reference circadian time;
a second series of steps that comprises:
selecting from the memory one or more second particular ones of the one or more data items, each respective one of the one or more second particular data items being representative of a respective second particular past circadian time at which the circadian time cycle had the same specific circadian phase as the specific circadian time;
for each respective one of the one or more second particular data items, determining the respective second particular past wall-clock time; and
generating a second contribution to the information based on a respective difference between the respective second particular wall-clock time and the specific wall-clock time; and
a graphical user-interface adapted to present the information generated by the data processor.

8. The system of claim 7, wherein
the data processor is configured for performing the first series of steps;
the specific circadian time is a current circadian time representative of a current circadian phase;
the specific wall-clock time is a current wall-clock time coinciding with the occurrence of the current circadian time; and
each respective particular past wall-clock time precedes the current wall-clock time by a respective integer number of wall-clock time-periods of 24-hours each.

9. The system of claim 7, wherein
the data processor is configured for performing the first series of steps;
the specific circadian time is a current circadian time representative of a current circadian phase;
the specific wall-clock time is a current wall-clock time coinciding with the occurrence of the current circadian time; and
each respective particular past wall-clock time precedes the current wall-clock time by a respective integer number of wall-clock time-periods of 168-hours each.

10. The system of claim 7, wherein:
the data processor is configured for performing the second series of steps;
the specific circadian phase is representative of a most recent occurrence of a pre-determined phase of the circadian time cycle;
the specific wall-clock time is a most recent wall-clock time coinciding with the most recent occurrence of the pre-determined phase; and
each respective second particular past circadian time is representative of a respective past occurrence of the pre-determined phase.

11. The system of claim 7, wherein:
the data processor is configured performing of the first series of steps;
the first series of steps comprises predicting a pattern, occurring in the future, of one or more stimuli that affect the person's circadian time cycle; and
the first contribution comprises a prediction of a future circadian time occurring at a future wall-clock time compatible with the specific wall-clock time.

12. The system of claim 7, wherein:
the data processor is configured for performing of the second series of steps;
the second series of steps comprises predicting a pattern, occurring in the future, of one or more stimuli that affect the person's circadian time cycle; and
the second contribution comprises a prediction of a future wall-clock time at which a future circadian time occurs compatible with the specific circadian time.

13. Control software (109) for configuring a computer (108) to present information about a person's circadian time cycle at a graphical user-interface (112), the control software comprising:
first instructions for determining, based on data from one or more light sensors and one or more motion sensors, a specific circadian time of the person that is representative of a specific circadian phase of the circadian time cycle;
second instructions for determining a specific wall-clock time coinciding with an occurrence of the specific circadian time;
third instructions for using a memory (106) that stores one or more data items, each respective one of the one or more data items is representative of a respective past circadian time of the person and is representative of a respective past wall-clock time coinciding with a respective past occurrence of the respective past circadian time;
at least one of: fourth instructions for performing a first series of steps and fifth instructions for performing a second series of steps;
the fourth instructions comprise:
sixth instructions for selecting from the memory one or more first particular ones of the one or more data items, each respective one of the one or more first particular data items being representative of a respective particular past wall-clock time that precedes the specific wall-clock time by a respective integer number of wall-clock time-periods of 24-hours;
seventh instructions for determining, for each respective one of the one or more particular data items, a respective particular past circadian time;
eighth instructions for determining a reference circadian time from the one or more particular past circadian times; and
ninth instructions for generating a first contribution to the information based on a difference between the specific circadian time and the reference circadian time;
the fifth instructions comprise:
tenth instructions for selecting from the memory one or more second particular ones of the one or more data items, each respective one of the one or more second particular data items being representative of a respective second particular past circadian time at which the circadian time cycle had the same specific circadian phase as the specific circadian time;
eleventh instructions for determining, for each respective one of the one or more second particular data items, the respective second particular past wall-clock time; and
twelfth instructions for generating a second contribution to the information based on a respective difference between the respective second particular wall-clock time and the specific wall-clock time; and
thirteenth instructions for presenting the information at a graphical user interface after having performed at least one of the fourth or fifth instructions.

14. The control software of claim 13, wherein the control software comprises the fourth instructions;
the specific circadian time is a current circadian time representative of a current circadian phase;
the specific wall-clock time is a current wall-clock time coinciding with the occurrence of the current circadian time; and
each respective particular past wall-clock time precedes the current wall-clock time by a respective integer number of wall-clock time-periods of 24-hours each.

15. The control software of claim 13, wherein
the control software comprises the fourth instructions;
the specific circadian time is a current circadian time representative of a current circadian phase;
the specific wall-clock time is a current wall-clock time coinciding with the occurrence of the current circadian time; and
each respective particular past wall-clock time precedes the current wall-clock time by a respective integer number of wall-clock time-periods of 168-hours each.

## Patentansprüche

1. Eine Methode zum Darstellen von Daten über den zirkadianen Zeitzyklus einer Person auf einer grafischen Benutzeroberfläche (112), wobei die Methode folgende Schritte umfasst:
beruhend auf den Daten mindestens eines Lichtsensors und mindestens eines Bewegungssensors Ermitteln der spezifischen zirkadianen Zeit der Person, die einer bestimmten zirkadianen Phase des zirkadianen Zeitzyklus entspricht;
Ermitteln einer bestimmten Wanduhrzeit, die mit dem Auftreten der spezifischen zirkadianen Zeit zusammenfällt;
Verwenden eines Speichers (106), auf dem mindestens ein Datenelement gespeichert wird, wobei jedes der mindestens einen Datenelemente jeweils der entsprechenden vergangenen zirkadianen Zeit für die Person sowie der jeweils vergangenen Wanduhrzeit entspricht, die mit einem jeweiligen vergangenen Auftreten der jeweiligen vergangenen zirkadianen Zeit zusammenfällt;
vor dem Darstellen der Daten auf einer graphischen Benutzeroberfläche Durchführen mindestens eines Schritts aus einer ersten Reihe von Schritten, die folgende Schritte umfasst:
Auswählen mindestens eines ersten bestimmten Datenelements der mehreren Datenelemente auf dem Speicher, wobei jedes der mindestens einen ersten bestimmten Datenelemente jeweils einer bestimmten vergangenen Wanduhrzeit entspricht, die der spezifischen Wanduhrzeit um jeweils eine ganzzahlige Anzahl von Wanduhr-Zeiträumen von 24 Stunden vorausgeht;
Ermitteln jeweils der bestimmten vergangenen zirkadianen Zeit für das mindestens eine der ersten entsprechenden Datenelemente;
Ermitteln einer zirkadianen Referenzzeit anhand der mindestens einen bestimmten vergangenen zirkadianen Zeiten; und
Erzeugen eines ersten Beitrags zu den Daten anhand der Differenz zwischen der spezifischen zirkadianen Zeit und der zirkadianen Referenzzeit;
eine zweite Reihe von Schritten, die folgende Schritte umfasst:
Auswählen mindestens eines zweiten bestimmten Datenelements der mehreren Datenelemente auf dem Speicher, wobei jedes der mindestens einen zweiten besonderen Datenelemente jeweils der zweiten entsprechenden vergangenen zirkadianen Zeit entspricht, zu der der zirkadiane Zeitzyklus dieselbe spezifische zirkadiane Phase aufwies, wie die spezifische zirkadiane Zeit;
Ermitteln jeweils der bestimmten zweiten vergangenen Wanduhrzeit für das mindestens eine der zweiten entsprechenden Datenelemente; und
Erzeugen eines zweiten Beitrags zu den Daten anhand der entsprechenden Differenz zwischen der entsprechenden zweiten Wanduhrzeit und der spezifischen Wanduhrzeit.

2. Die Methode gemäß Anspruch 1, wobei die Methode das Durchführen der ersten Reihe von Schritten umfasst;
und wobei es sich bei der spezifischen zirkadianen Zeit um die aktuelle zirkadiane Zeit handelt, die der aktuellen zirkadianen Phase entspricht;
und wobei es sich bei der spezifischen Wanduhrzeit um die aktuelle Wanduhrzeit handelt, die mit dem Auftreten der aktuellen zirkadianen Zeit zusammenfällt; und
wobei die einzelnen entsprechenden vergangenen Wanduhrzeiten der aktuellen Wanduhrzeit um eine jeweils ganzzahlige Anzahl von Wanduhr-Zeiträumen von jeweils 24 Stunden vorausgehen.

3. Die Methode gemäß Anspruch 1, wobei die Methode das Durchführen der ersten Reihe von Schritten umfasst;
und wobei es sich bei der spezifischen zirkadianen Zeit um die aktuelle zirkadiane Zeit handelt, die der aktuellen zirkadianen Phase entspricht;
und wobei es sich bei der spezifischen Wanduhrzeit um die aktuelle Wanduhrzeit handelt, die mit dem Auftreten der aktuellen zirkadianen Zeit zusammenfällt; und
wobei die einzelnen entsprechenden vergangenen Wanduhrzeiten der aktuellen Wanduhrzeit um eine jeweils ganzzahlige Anzahl von Wanduhr-Zeiträumen von jeweils 168 Stunden vorausgehen.

4. Die Methode gemäß Anspruch 1, wobei:
die Methode das Durchführen der zweiten Reihe von Schritten umfasst;
die spezifische zirkadiane Phase dem aktuellsten Auftreten einer vorab festgelegten Phase des zirkadianen Zeitzyklus entspricht;
es sich bei der spezifischen Wanduhrzeit um die aktuellste Wanduhrzeit handelt, die mit dem aktuellsten Auftreten der vorab festgelegten Phase zusammenfällt; und
die einzelnen entsprechenden zweiten bestimmten vergangenen zirkadianen Zeiten dem jeweiligen vergangenen Auftreten der vorab festgelegten Phase entsprechen.

5. Die Methode gemäß Anspruch 1,wobei:
die Methode das Durchführen der ersten Reihe von Schritten umfasst;
die erste Reihe von Schritten das Vorhersagen eines zukünftigen Musters für mindestens einen Reiz umfasst, der den zirkadianen Zeitzyklus der Person beeinflusst;
der erste Beitrag eine Vorhersage der zukünftigen zirkadianen Zeit umfasst, die zu einer zukünftigen Wanduhrzeit eintritt, die mit der spezifischen Wanduhrzeit abgestimmt ist.

6. Die Methode gemäß Anspruch 1, wobei:
die Methode das Durchführen der zweiten Reihe von Schritten umfasst;
die zweite Reihe von Schritten das Vorhersagen eines zukünftigen Musters für mindestens einen Reiz umfasst, der den zirkadianen Zeitzyklus der Person beeinflusst;
der zweite Beitrag eine Vorhersage der zukünftigen Wanduhrzeit umfasst, zu der die zukünftige zirkadiane Zeit eintritt, und die mit der spezifischen zirkadianen Zeit abgestimmt ist.

7. Ein System (100) zum Darstellen von Daten über den zirkadianen Zeitzyklus einer Person auf einer grafischen Benutzeroberfläche (112), wobei das System Folgendes umfasst:
ein in die Hardware und/oder Software integriertes zirkadianes Modul (102), das beruhend auf den Daten mindestens eines Lichtsensors und mindestens eines Bewegungssensors die spezifische zirkadiane Zeit der Person ermittelt, die einer bestimmten zirkadianen Phase des zirkadianen Zeitzyklus entspricht;
eine Uhr zum Ermitteln einer bestimmten Wanduhrzeit, die mit dem Auftreten der spezifischen zirkadianen Zeit zusammenfällt;
einen Speicher (106), auf dem mindestens ein Datenelement gespeichert wird, wobei jedes der mindestens einen Datenelemente jeweils der entsprechenden vergangenen zirkadianen Zeit für die Person sowie der jeweils vergangenen Wanduhrzeit entspricht, die mit einem jeweiligen vergangenen Auftreten der jeweiligen vergangenen zirkadianen Zeit zusammenfällt; und
einen Datenprozessor (108), der mindestens eines der Folgenden ausführt:
eine erste Reihe von Schritten, die folgende Schritte umfasst:
Auswählen mindestens eines ersten bestimmten Datenelements der mehreren Datenelemente auf dem Speicher, wobei jedes der mindestens einen ersten bestimmten Datenelemente jeweils einer bestimmten vergangenen Wanduhrzeit entspricht, die der spezifischen Wanduhrzeit um jeweils eine ganzzahlige Anzahl von Wanduhr-Zeiträumen von 24 Stunden vorausgeht;
Ermitteln jeweils der bestimmten vergangenen zirkadianen Zeit für das mindestens eine der ersten entsprechenden Datenelemente;
Ermitteln einer zirkadianen Referenzzeit anhand der mindestens einen bestimmten vergangenen zirkadianen Zeiten; und
Erzeugen eines ersten Beitrags zu den Daten anhand der Differenz zwischen der spezifischen zirkadianen Zeit und der zirkadianen Referenzzeit;
eine zweite Reihe von Schritten, die folgende Schritte umfasst:
Auswählen mindestens eines zweiten bestimmten Datenelements der mehreren Datenelemente auf dem Speicher, wobei jedes der mindestens einen zweiten besonderen Datenelemente jeweils der zweiten entsprechenden vergangenen zirkadianen Zeit entspricht, zu der der zirkadiane Zeitzyklus dieselbe spezifische zirkadiane Phase aufwies, wie die spezifische zirkadiane Zeit;
Ermitteln jeweils der bestimmten zweiten vergangenen Wanduhrzeit für das mindestens eine der zweiten entsprechenden Datenelemente; und
Erzeugen eines zweiten Beitrags zu den Daten anhand der entsprechenden Differenz zwischen der entsprechenden zweiten Wanduhrzeit und der spezifischen Wanduhrzeit; und
eine grafische Benutzeroberfläche zum Darstellen der vom Datenprozessor generierten Daten.

8. Das System gemäß Anspruch 7, wobei der Datenprozessor die erste Reihe von Schritten ausführt;
und wobei es sich bei der spezifischen zirkadianen Zeit um die aktuelle zirkadiane Zeit handelt, die der aktuellen zirkadianen Phase entspricht;
und wobei es sich bei der spezifischen Wanduhrzeit um die aktuelle Wanduhrzeit handelt, die mit dem Auftreten der aktuellen zirkadianen Zeit zusammenfällt; und
wobei die einzelnen entsprechenden vergangenen Wanduhrzeiten der aktuellen Wanduhrzeit um eine jeweils ganzzahlige Anzahl von Wanduhr-Zeiträumen von jeweils 24 Stunden vorausgehen.

9. Das System gemäß Anspruch 7, wobei der Datenprozessor die erste Reihe von Schritten ausführt;
und wobei es sich bei der spezifischen zirkadianen Zeit um die aktuelle zirkadiane Zeit handelt, die der aktuellen zirkadianen Phase entspricht;
und wobei es sich bei der spezifischen Wanduhrzeit um die aktuelle Wanduhrzeit handelt, die mit dem Auftreten der aktuellen zirkadianen Zeit zusammenfällt; und
wobei die einzelnen entsprechenden vergangenen Wanduhrzeiten der aktuellen Wanduhrzeit um eine jeweils ganzzahlige Anzahl von Wanduhr-Zeiträumen von jeweils 168 Stunden vorausgehen.

10. Das System gemäß Anspruch 7, wobei:
der Datenprozessor die zweite Reihe von Schritten ausführt;
die spezifische zirkadiane Phase dem aktuellsten Auftreten einer vorab festgelegten Phase des zirkadianen Zeitzyklus entspricht;
es sich bei der spezifischen Wanduhrzeit um die aktuellste Wanduhrzeit handelt, die mit dem aktuellsten Auftreten der vorab festgelegten Phase zusammenfällt; und
die einzelnen entsprechenden zweiten bestimmten vergangenen zirkadianen Zeiten dem jeweiligen vergangenen Auftreten der vorab festgelegten Phase entsprechen.

11. Das System gemäß Anspruch 7, wobei:
der Datenprozessor die erste Reihe von Schritten ausführt;
die erste Reihe von Schritten das Vorhersagen eines zukünftigen Musters für mindestens einen Reiz umfasst, der den zirkadianen Zeitzyklus der Person beeinflusst;
der erste Beitrag eine Vorhersage der zukünftigen zirkadianen Zeit umfasst, die zu einer zukünftigen Wanduhrzeit eintritt, die mit der spezifischen Wanduhrzeit abgestimmt ist.

12. Das System gemäß Anspruch 7, wobei:
der Datenprozessor die zweite Reihe von Schritten ausführt;
die zweite Reihe von Schritten das Vorhersagen eines zukünftigen Musters für mindestens einen Reiz umfasst, der den zirkadianen Zeitzyklus der Person beeinflusst;
der zweite Beitrag eine Vorhersage der zukünftigen Wanduhrzeit umfasst, zu der die zukünftige zirkadiane Zeit eintritt, und die mit der spezifischen zirkadianen Zeit abgestimmt ist.

13. Steuerungssoftware (109) zum Konfigurieren eines Computers (108) für das Darstellen von Daten über den zirkadianen Zeitzyklus einer Person auf einer grafischen Benutzeroberfläche (112), wobei die Steuerungssoftware Folgendes umfasst:
erste Anweisungen, um beruhend auf den Daten mindestens eines Lichtsensors und mindestens eines Bewegungssensors die spezifische zirkadiane Zeit der Person zu ermitteln, die einer bestimmten zirkadianen Phase des zirkadianen Zeitzyklus entspricht;
zweite Anweisungen zum Ermitteln einer bestimmten Wanduhrzeit, die mit dem Auftreten der spezifischen zirkadianen Zeit zusammenfällt;
dritte Anweisungen zum Verwenden eines Speichers (106), auf dem mindestens ein Datenelement gespeichert wird, wobei jedes der mindestens einen Datenelemente jeweils der entsprechenden vergangenen zirkadianen Zeit für die Person sowie der jeweils vergangenen Wanduhrzeit entspricht, die mit einem jeweiligen vergangenen Auftreten der jeweiligen vergangenen zirkadianen Zeit zusammenfällt;
mindestens eines der Folgenden: vierte Anweisungen zum Durchführen einer ersten Reihe von Schritten und
fünfte Anweisungen zum Durchführen einer zweiten Reihe von Schritten;
wobei die vierten Anweisungen Folgendes umfassen:
sechste Anweisungen zum Auswählen mindestens eines ersten bestimmten Datenelements der mehreren Datenelemente auf dem Speicher, wobei jedes der mindestens einen ersten bestimmten Datenelemente jeweils einer bestimmten vergangenen Wanduhrzeit entspricht, die der spezifischen Wanduhrzeit um jeweils eine ganzzahlige Anzahl von Wanduhr-Zeiträumen von 24 Stunden vorausgeht;
siebte Anweisungen zum Ermitteln jeweils der bestimmten vergangenen zirkadianen Zeit für das mindestens eine der entsprechenden Datenelemente;
achte Anweisungen zum Ermitteln einer zirkadianen Referenzzeit anhand der mindestens einen bestimmten vergangenen zirkadianen Zeiten; und
neunte Anweisungen zum Erzeugen eines ersten Beitrags zu den Daten anhand der Differenz zwischen der spezifischen zirkadianen Zeit und der zirkadianen Referenzzeit;
wobei die fünften Anweisungen Folgendes umfassen:
zehnte Anweisungen zum Auswählen mindestens eines zweiten bestimmten Datenelements der mehreren Datenelemente auf dem Speicher, wobei jedes der mindestens einen zweiten besonderen Datenelemente jeweils der zweiten entsprechenden vergangenen zirkadianen Zeit entspricht, zu der der zirkadiane Zeitzyklus dieselbe spezifische zirkadiane Phase aufwies, wie die spezifische zirkadiane Zeit;
elfte Anweisungen zum Ermitteln jeweils der bestimmten zweiten vergangenen Wanduhrzeit für das mindestens eine der zweiten entsprechenden Datenelemente; und
zwölfte Anweisungen zum Erzeugen eines zweiten Beitrags zu den Daten anhand der entsprechenden Differenz zwischen der entsprechenden zweiten Wanduhrzeit und der spezifischen Wanduhrzeit; und
dreizehnte Anweisungen zum Darstellen der Daten auf einer grafischen Benutzeroberfläche, nachdem mindestens jeweils die vierte oder fünfte Anweisung ausgeführt wurde.

14. Die Steuerungssoftware gemäß Anspruch 13, wobei die Steuerungssoftware die vierten Anweisungen umfasst;
und wobei es sich bei der spezifischen zirkadianen Zeit um die aktuelle zirkadiane Zeit handelt, die der aktuellen zirkadianen Phase entspricht;
und wobei es sich bei der spezifischen Wanduhrzeit um die aktuelle Wanduhrzeit handelt, die mit dem Auftreten der aktuellen zirkadianen Zeit zusammenfällt; und
wobei die einzelnen entsprechenden vergangenen Wanduhrzeiten der aktuellen Wanduhrzeit um eine jeweils ganzzahlige Anzahl von Wanduhr-Zeiträumen von jeweils 24 Stunden vorausgehen.

15. Die Steuerungssoftware gemäß Anspruch 13,wobei die Steuerungssoftware die vierten Anweisungen umfasst;
und wobei es sich bei der spezifischen zirkadianen Zeit um die aktuelle zirkadiane Zeit handelt, die der aktuellen zirkadianen Phase entspricht;
und wobei es sich bei der spezifischen Wanduhrzeit um die aktuelle Wanduhrzeit handelt, die mit dem Auftreten der aktuellen zirkadianen Zeit zusammenfällt; und wobei die einzelnen entsprechenden vergangenen Wanduhrzeiten der aktuellen Wanduhrzeit um eine jeweils ganzzahlige Anzahl von Wanduhr-Zeiträumen von jeweils 168 Stunden vorausgehen.

## Revendications

1. Procédé de présentation des informations relatives à la durée du cycle circadien d'une personne à une interface utilisateur graphique (112), le procédé comprenant :
la détermination, sur la base de données provenant d'un ou de plusieurs capteurs de lumière et d'un ou de plusieurs capteurs de mouvement, d'un temps circadien spécifique de la personne qui est représentatif d'une phase circadienne spécifique de la durée du cycle circadien ;
la détermination d'un temps d'horloge murale spécifique coïncidant avec une occurrence du temps circadien spécifique ;
l'utilisation d'une mémoire (106) qui stocke un ou plusieurs éléments de données, chaque élément de données respectif d'un ou de plusieurs éléments de données est représentatif d'un temps circadien passé respectif de la personne et est représentatif d'un temps d'horloge murale passé respectif coïncidant avec une occurrence passée respective du temps circadien passé spécifique ;
avant présentation des informations sur une interface utilisateur graphique, réalisation d'au moins :
une première série d'étapes qui comprend :
la sélection à partir de la mémoire d'un ou de plusieurs premiers éléments de données particuliers, chaque élément de données respectif d'un ou de plusieurs premiers éléments de données particuliers étant représentatif d'un temps d'horloge murale passé particulier respectif qui précède le temps d'horloge murale spécifique d'un nombre entier respectif de périodes de temps d'horloge murale de 24 heures;
pour chaque élément de données respectif d'un ou de plusieurs premiers éléments de données particuliers, détermination d'un temps circadien passé particulier respectif;
détermination d'un temps circadien de référence à partir d'un ou de plusieurs temps circadiens passés particuliers; et
génération d'une première contribution aux informations basée sur une différence entre le temps circadien spécifique et le temps circadien de référence;
une seconde série d'étapes qui comprend:
la sélection à partir de la mémoire d'un ou de plusieurs seconds éléments de données particuliers d'un ou de plusieurs éléments de données, chaque élément de données respectif d'un ou de plusieurs seconds éléments de données particuliers étant représentatif d'un second temps circadien passé particulier respectif auquel la durée du cycle circadien avait la même phase circadienne spécifique que le temps circadien spécifique;
pour chaque élément de données respectif d'un ou de plusieurs seconds éléments de données particuliers, détermination du second temps d'horloge murale passé particulier respectif; et
génération d'une seconde contribution aux informations sur la base d'une différence respective entre le second temps d'horloge murale particulier respectif et le temps d'horloge murale spécifique.

2. Procédé selon la revendication 1, dans lequel le procédé comprend la réalisation des premières séries d'étapes;
le temps circadien spécifique est un temps circadien actuel représentatif d'une phase circadienne actuelle; le temps d'horloge murale spécifique est un temps d'horloge murale actuelle coïncidant avec l'occurrence du temps circadien actuel; et
chaque temps d'horloge murale passé spécifique respectif précède le temps d'horloge murale actuel d'un nombre entier respectif de périodes de temps d'horloge murale de 24 heures chacune.

3. Procédé selon la revendication 1, dans lequel :
le procédé comprend la réalisation de la première série d'étapes;
le temps circadien spécifique est un temps circadien actuel représentatif d'une phase circadienne actuelle;
le temps d'horloge murale spécifique est un temps d'horloge actuel coïncidant avec l'occurrence du temps circadien actuel; et
chaque temps d'horloge murale passé particulier respectif précède le temps d'horloge murale actuel d'un nombre entier respectif de périodes de temps d'horloge murale de 168 heures chacune.

4. Procédé selon la revendication 1, dans lequel :
le procédé comprend la réalisation de la seconde série d'étapes;
la phase circadienne spécifique est représentative d'une occurrence plus récente d'une phase prédéterminée de la durée du cycle circadien;
le temps d'horloge spécifique est un temps d'horloge plus récent coïncidant avec l'occurrence la plus récente de la phase prédéterminée; et
chaque second temps circadien passé particulier respectif est représentatif d'une occurrence passée respective de la phase prédéterminée.

5. Procédé selon la revendication 1, dans lequel :
le procédé comprend la réalisation de la première série d'étapes;
la première série d'étapes comprend la prédiction d'un futur modèle d'un ou de plusieurs stimuli qui affectent la durée du cycle circadien de la personne;
la première contribution comprend une prédiction d'un temps circadien futur survenant à un temps d'horloge murale futur compatible avec le temps d'horloge murale spécifique.

6. Procédé selon la revendication 1, dans lequel :
le procédé comprend la réalisation de la seconde série d'étapes;
la seconde série d'étapes comprend la prédiction d'un futur modèle d'un ou de plusieurs stimuli qui affectent la durée du cycle circadien de la personne;
la seconde contribution comprend une prédiction d'un temps d'horloge murale futur auquel un temps circadien futur apparaît compatible avec le temps circadien spécifique.

7. Système (100) configuré pour présenter des informations relatives à la durée du cycle circadien d'une personne à une interface utilisateur graphique (112), le système comprenant:
un moteur circadien (102), implémenté dans un matériel et/ou logiciel, configuré pour déterminer, sur la base des données d'un ou de plusieurs capteurs de lumière et d'un ou de plusieurs capteurs de mouvement,
un temps circadien spécifique de la personne qui est représentatif d'une phase circadienne de la durée du cycle circadien;
une horloge configurée pour déterminer un temps d'horloge murale spécifique coïncidant avec une occurrence du temps circadien spécifique;
une mémoire (106) qui stocke un ou plusieurs éléments de données, chaque élément de données respectif d'un ou de plusieurs éléments de données est représentatif d'un temps circadien spécifique de la personne et est représentatif d'un temps d'horloge murale passé respectif coïncidant avec une occurrence passée respective du temps circadien passé respectif ; et
un processeur de données (108) configuré pour réaliser au moins:
une première série d'étapes qui comprend:
la sélection à partir de la mémoire d'un ou de plusieurs premiers éléments de données particuliers, chaque élément de données respectif d'un ou de plusieurs premiers éléments de données particuliers étant représentatif d'un temps d'horloge murale passé particulier respectif qui précède le temps d'horloge murale spécifique d'un nombre entier respectif de périodes de temps d'horloge murale de 24 heures;
pour chaque élément de données respectif d'un ou de plusieurs premiers éléments de données particuliers, détermination d'un temps circadien passé particulier respectif;
détermination d'un temps circadien de référence à partir d'un ou de plusieurs temps circadiens passés particuliers; et
génération d'une première contribution aux informations sur la base d'une différence entre le temps circadien spécifique et le temps circadien de référence;
une seconde série d'étapes qui comprend:
la sélection à partir de la mémoire d'un ou de plusieurs seconds éléments de données particuliers d'un ou de plusieurs éléments de données, chaque élément de données respectif d'un ou de plusieurs seconds éléments de données particuliers étant représentatif d'un second temps circadien passé particulier respectif auquel la durée du cycle circadien avait la même phase circadienne spécifique que le temps circadien spécifique;
pour chaque élément de données respectif d'un ou de plusieurs seconds éléments de données particuliers, détermination du second temps d'horloge murale passé particulier respectif; et
génération d'une seconde contribution aux informations sur la base d'une différence respective entre le second temps d'horloge murale particulier respectif et le temps d'horloge murale spécifique; et
une interface utilisateur graphique conçue pour présenter les informations générées par le processeur de données.

8. Système selon la revendication 7, dans lequel le processeur de données est configuré pour réaliser la première série d'étapes;
le temps circadien spécifique est un temps circadien actuel représentatif d'une phase circadienne actuelle; le temps d'horloge murale spécifique est un temps d'horloge murale actuel coïncidant avec l'occurrence du temps circadien actuel; et
chaque temps d'horloge murale passé particulier respectif précède le temps d'horloge murale actuel d'un nombre entier respectif de périodes de temps d'horloge murale de 24 heures chacune.

9. Système selon la revendication 7, dans lequel le processeur de données est configuré pour réaliser les premières séries d'étapes;
le temps circadien spécifique est un temps circadien actuel représentatif d'une phase circadienne actuelle; le temps d'horloge murale spécifique est un temps d'horloge murale actuel coïncidant avec l'occurrence du temps circadien actuel; et
chaque temps d'horloge murale passé particulier respectif précède le temps d'horloge murale actuel d'un nombre entier respectif de périodes de temps d'horloge murale de 168 heures chacune.

10. Système selon la revendication 7, dans lequel:
le processeur de données est configuré pour réaliser les secondes séries d'étapes;
la phase circadienne spécifique est représentative d'une occurrence plus récente d'une phase prédéterminée de la durée du cycle circadien;
le temps d'horloge murale spécifique est un temps d'horloge murale plus récent coïncidant avec l'occurrence plus récente de la phase prédéterminée; et
chaque second temps circadien passé particulier respectif est représentatif d'une occurrence passée respective de la phase prédéterminée.

11. Système selon la revendication 7, dans lequel:
le processeur de données est configuré pour réaliser les premières séries d'étapes;
les premières séries d'étapes comprennent la prédiction d'un modèle, se produisant dans le futur, d'un ou de plusieurs stimuli qui affectent la durée du cycle circadien de la personne; et
la première contribution comprend une prédiction du temps circadien futur se produisant à un temps d'horloge murale futur compatible avec le temps d'horloge murale spécifique.

12. Système selon la revendication 7, dans lequel:
le processeur de données est configuré pour réaliser les secondes séries d'étapes;
les secondes séries d'étapes comprennent la prédiction d'un modèle, se produisant dans le futur, d'un ou de plusieurs stimuli qui affectent la durée du cycle circadien de la personne; et
la seconde contribution comprend une prédiction du temps circadien futur survenant à un temps d'horloge murale futur compatible avec le temps circadien spécifique.

13. Logiciel de contrôle (109) pour configurer un ordinateur (108) afin de présenter des informations sur une durée de cycle circadien d'une personne à une interface graphique utilisateur (112), le logiciel de contrôle comprenant:
des premières instructions pour déterminer, sur la base de données provenant d'un ou de plusieurs capteurs de lumière et d'un ou de plusieurs capteurs de mouvement, un temps circadien spécifique de la personne qui est représentatif d'une phase circadienne spécifique de la durée du cycle circadien;
des secondes instructions pour déterminer un temps d'horloge murale spécifique coïncidant avec une occurrence du temps circadien spécifique;
des troisièmes instructions pour utiliser une mémoire (106) qui stocke un ou plusieurs éléments de données, chaque élément de données respectif d'un ou de plusieurs éléments de données est représentatif d'un temps circadien passé respectif de la personne et est représentatif d'un temps d'horloge murale passé respectif coïncidant avec une occurrence passée respective du temps circadien passé spécifique;
au moins l'une des : quatrièmes instructions pour réaliser une première série d'étapes et cinquièmes instructions pour réaliser une seconde série d'étapes;
les quatrièmes instructions comprennent:
les sixièmes instructions pour sélectionner à partir de la mémoire d'un ou de plusieurs premiers éléments de données particuliers, chaque élément de données respectif d'un ou de plusieurs premiers éléments de données particuliers étant représentatif d'un temps d'horloge murale passé particulier respectif qui précède le temps d'horloge murale spécifique d'un nombre entier respectif de périodes de temps d'horloge murale de 24 heures;
les septièmes instructions pour déterminer, pour chaque élément de données respectif d'un ou de plusieurs éléments de données particuliers, un temps circadien passé particulier respectif;
les huitièmes instructions pour déterminer un temps circadien de référence à partir d'un ou de plusieurs temps circadiens passés particuliers; et
les neuvièmes instructions pour générer une première contribution aux informations sur la base d'une différence entre le temps circadien spécifique et le temps circadien de référence;
les cinquièmes instructions comprennent:
les dixièmes instructions pour sélectionner à partir de la mémoire d'un ou de plusieurs seconds éléments de données particuliers, chaque élément de données respectif d'un ou de plusieurs seconds éléments de données particuliers étant représentatif d'un second temps circadien passé particulier auquel la durée de cycle circadien avait la même phase circadienne spécifique que le temps circadien spécifique;
les onzièmes instructions pour déterminer, pour chaque élément de données d'un ou de plusieurs seconds éléments de données particuliers, le second temps d'horloge murale passé particulier respectif ; et
les douzièmes instructions pour générer une seconde contribution aux informations sur la base d'une différence respective entre le second temps d'horloge murale particulier respectif et le temps d'horloge murale spécifique; et
les treizièmes instructions pour présenter les informations à une interface utilisateur graphique après avoir réalisé au moins l'une des quatrièmes ou cinquièmes instructions.

14. Logiciel de contrôle selon la revendication 13, dans lequel le logiciel de contrôle comprend les quatrièmes instructions;
le temps circadien spécifique est un temps circadien actuel représentatif d'une phase circadienne actuelle; le temps d'horloge murale spécifique est un temps d'horloge murale actuel coïncidant avec l'occurrence de temps circadien actuel; et
chaque temps d'horloge murale passé particulier respectif précède le temps d'horloge murale spécifique d'un nombre entier respectif de périodes de temps d'horloge murale de 24 heures chacune.

15. Logiciel de contrôle selon la revendication 13, dans lequel le logiciel de contrôle comprend les quatrièmes instructions;
le temps circadien spécifique est un temps circadien actuel représentatif d'une phase circadienne actuelle; le temps d'horloge murale spécifique est un temps d'horloge murale coïncidant avec l'occurrence du temps circadien actuel; et
chaque temps particulier d'horloge murale passé respectif précède le temps d'horloge murale actuel d'un nombre entier respectif de périodes de temps d'horloge murale de 168 heures chacune.
